# EUROPEAN PATENT APPLICATION

(11) **EP 2 806 369 A2**
(43) Date of publication of application: **26.11.2014**
(21) Application number: 14169651.8
(22) Date of filing: 23.05.2014
(51) Int. Cl.: G06F 19/00

(54) **Medical device implant monitoring**

(30) Priority: 24.05.2013 US 201361827308 P
(71) Applicant: Spang, Angela, Minnetonka, MN 55343 (US)
(72) Inventor: Spang, Angela, Minnetonka, MN 55343 (US)
(74) Representative: Vossius & Partner

(57) **Abstract**

In a method of monitoring complications relating to a plurality of medical devices, which are each implanted in one of a plurality of patients, information for each of the implanted medical devices is entered in a computing device (102) by displaying (170) a plurality of candidate complications for the medical device on a display (108) of the computing device, receiving (172) a selection of one or more of the candidate complications using an input device (110) of the computing device, and storing (174) the one or more selected candidate complications a memory of the computing device or a remote data store (106, 114, 120). One or more reports identifying the selected complications for the medical devices are generated (176) using the computing device, and the one or more reports are displayed (178) on the display.

## Description

### FIELD

Embodiments of the invention are directed to methods and devices for monitoring complications relating to medical devices that have been implanted in patients. In some embodiments, the medical devices are configured to treat a pelvic condition of the patient.

### BACKGROUND

Devices are commonly implanted in patients to treat a condition of the patient. Some implantable medical devices are designed to treat pelvic conditions, such as urinary incontinence, pelvic organ prolapse, fecal incontinence, urine retention, sexual dysfunction, and pelvic pain, for example. Such devices are implanted by physicians all over the world.

Complications may arise both during and after the implantation of the medical devices. Unfortunately, such incidents are not reported in a manner that would allow for statistical insight into the incidence and severity of such complications in a group of patients in which a device has been implanted.

### SUMMARY

Some embodiments of the invention are directed to a method of monitoring complications relating to a plurality of medical devices, which are each implanted in one of a plurality of patients. In the method, complication information for each of the implanted medical devices is entered in a computing device. In some embodiments, this involves displaying a plurality of candidate complications for the medical device on a display of the computing device, receiving a selection of one or more of the candidate complications using an input device of the computing device, and storing the one or more selected candidate complications a memory of the computing device or a remote data store. One or more reports identifying the selected complications for the medical devices are generated using the computing device, and the one or more reports are displayed on the display.

Some embodiments are directed to a computing device for monitoring complications relating to a plurality of medical devices, which are each implanted in one of a plurality of patients. In some embodiments, the computing device includes a processor, memory comprising a non-transitory computer readable medium, a display, and an input device. Instructions are stored in the non-transitory computer readable medium, which when executed by the processor causes the processor to perform operations for monitoring complications relating to the medical device implantations. In some embodiments, the operations include entering complication information for each of the implanted medical devices. In some embodiments, this operation involves displaying a plurality of candidate complications for the medical device on the display, receiving a selection of one or more candidate complications corresponding to the medical device from the input device, and storing the one or more selected candidate complications in at least one of the memory of the computing device and a remote data store. In some embodiments, the operations also include generating one or more reports identifying the selected complications for the medical devices, and displaying the report on the display.

In some embodiments, the medical devices are configured to treat a pelvic condition of the patient selected from the group consisting of urinary incontinence, pelvic organ prolapse, fecal incontinence, urine retention, sexual dysfunction, and pelvic pain. In some embodiments, the medical devices are each selected from the group consisting of a sling, an artificial sphincter, a penile prosthesis, and an electronic stimulator device.

In some embodiments, the input device includes at least one input device selected from the group consisting of a keypad, a speech recognition system, a touchpad, and a touchscreen.

In some embodiments, the candidate complications are selected from the group consisting of vascular injury, visceral injury, neural injury, pain, infection, dysfunction, excessive bleeding, bladder cap tamponade, thrombosis, embolism, lymphocele, visceral injury to the bladder, visceral rectal injury, visceral bowel injury, visceral injury to the vaginal wall, visceral injury to the perineum, visceral injury to the urethra, visceral injury to the ureter, loss of feeling, neuropathy, perineum pain, dysuria, dyspareunia, leg pain, genital pain, fever, urinary tract infection, wound dehiscence, necrosis, increased incontinence, increased vaginal prolapse, and urinary tension.

In some embodiments, the method step and operation of entering complication information for each of the implanted medical devices comprise entering a score for each of the selected candidate complications using the input device. In some embodiments, the score is indicative of a severity of the complication.

In some embodiments, the plurality of medical devices comprises first and second medical devices, and generating one or more reports identifying the selected complications for the medical devices using the computing device comprises generating a report comprising a summary of the received selected complications corresponding to the first medical devices, and generating a report comprising a summary of the received selected complications corresponding to the second medical devices.

In some embodiments, the method steps and operations performed by the processor in response to the execution of instructions stored on the computer readable medium include entering patient information for each of the patients in the computing device using the input device, the patient information including at least two pieces of information selected from the group consisting of an identification of the patient, an identification of a surgical procedure performed on the patient to implant the medical device, and an identification of the medical device implanted in the patient. In some embodiments, the operation of generating one or more reports identifying the selected complications for the medical devices comprises generating a patient report, such as the patient summary screen for one of the patients that includes the patient information and the selected complications corresponding to the medical device implanted in the patient.

Some embodiments of the method steps and operations comprise generating a timeline screen listing a plurality of surgeries that were performed to implant the medical devices on the display, wherein the listing includes an alert icon indicating that one or more complications relating to at least one of the surgeries were observed.

This Summary is provided to introduce a selection of concepts in a simplified form that are further described below in the Detailed Description. This Summary is not intended to identify key features or essential features of the claimed subject matter, nor is it intended to be used as an aid in determining the scope of the claimed subject matter. The claimed subject matter is not limited to implementations that solve any or all disadvantages noted in the Background.

### BRIEF DESCRIPTION OF THE DRAWINGS

FIG. 1 is a simplified block diagram of a system and computing device for monitoring complications relating to medical devices that have been implanted in patients, in accordance with embodiments of the invention.
FIGS. 2-19 illustrate exemplary screens presented on a display of a computing device, in accordance with embodiments of the invention.
FIG. 20 is a flowchart illustrating a method of monitoring complications relating to a plurality of medical devices, which are each implanted in one of a plurality of patients, in accordance with embodiments of the invention.

### DETAILED DESCRIPTION OF ILLUSTRATIVE EMBODIMENTS

Embodiments of the invention are described more fully hereinafter with reference to the accompanying drawings. Elements that are identified using the same or similar reference characters refer to the same or similar elements. The various embodiments of the invention may, however, be embodied in many different forms and should not be construed as limited to the embodiments set forth herein. Rather, these embodiments are provided so that this disclosure will be thorough and complete, and will fully convey the scope of the invention to those skilled in the art.

Specific details are given in the following description to provide a thorough understanding of the embodiments. However, it is understood by those of ordinary skill in the art that the embodiments may be practiced without these specific details. For example, circuits, systems, networks, processes, frames, supports, connectors, motors, processors, and other components may not be shown, or shown in block diagram form in order to not obscure the embodiments in unnecessary detail.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

It will be understood that when an element is referred to as being "connected" or "coupled" to another element, it can be directly connected or coupled to the other element or intervening elements may be present. In contrast, if an element is referred to as being "directly connected" or "directly coupled" to another element, there are no intervening elements present.

It will be understood that, although the terms first, second, etc. may be used herein to describe various elements, these elements should not be limited by these terms. These terms are only used to distinguish one element from another. Thus, a first element could be termed a second element without departing from the teachings of the present invention.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

As will further be appreciated by one of skill in the art, the present invention may be embodied as methods, systems, and/or computer program products. Accordingly, the present invention may take the form of an entirely hardware embodiment, an entirely software embodiment or an embodiment combining software and hardware aspects. The computer program or software aspect of the present invention may comprising computer readable instructions or code stored in a computer readable medium or memory. Execution of the program instructions by one or more processors (e.g., central processing unit) results in the one or more processors performing one or more functions, method steps or operations described herein. Any suitable patent subject matter eligible computer readable media or memory may be utilized including, for example, hard disks, CD-ROMs, optical storage devices, or magnetic storage devices. Such computer readable media or memory do not include transitory waves or signals.

The computer-usable or computer-readable medium may be, for example but not limited to, an electronic, magnetic, optical, electromagnetic, infrared, or semiconductor system, apparatus, device, or propagation medium. More specific examples (a non-exhaustive list) of the computer-readable medium would include the following: an electrical connection having one or more wires, a portable computer diskette, a random access memory (RAM), a read-only memory (ROM), an erasable programmable read-only memory (EPROM or Flash memory), an optical fiber, and a portable compact disc read-only memory (CD-ROM). Note that the computer-usable or computer-readable medium could even be paper or another suitable medium upon which the program is printed, as the program can be electronically captured, via, for instance, optical scanning of the paper or other medium, then compiled, interpreted, or otherwise processed in a suitable manner, if necessary, and then stored in a computer memory.

Embodiments of the invention may also be described using flowchart illustrations and block diagrams. Although a flowchart may describe the operations as a sequential process, many of the operations can be performed in parallel or concurrently. In addition, the order of the operations may be re-arranged. A process is terminated when its operations are completed, but could have additional steps not included in a figure or described herein.

It is understood that one or more of the blocks (of the flowcharts and block diagrams) may be implemented by computer program instructions. These program instructions may be provided to a processor circuit, such as a microprocessor, microcontroller or other processor, which executes the instructions to implement the functions specified in the block or blocks through a series of operational steps to be performed by the processor(s) and corresponding hardware components.

Embodiments of the invention are directed to a system for monitoring or information regarding implantable medical devices (IMD's), such as those used to treat pelvic conditions. In some embodiments, the objective is to track or monitor IMD's rather than the patients. This allows usage across country borders and different hospital systems.

Exemplary pelvic conditions include urinary incontinence, pelvic organ prolapse, fecal incontinence, urine retention, sexual dysfunction (e.g., impotence), and pelvic pain. Exemplary IMD's used to treat pelvic conditions include slings or tapes used to support the pelvic floor, the urethra or other pelvic organ or musculature, an artificial sphincter used to close a lumen of the patient (e.g., urethra), an electronic stimulator device used to apply electrical stimulation waveforms to a pelvic organ or musculature, a penile prosthesis, or other implantable medical device that is configured to treat a pelvic condition of the patient. Examples of such IMD's are disclosed in the following U.S. patents, which are incorporated herein by reference in their entirety: 6,896,651; 8,195,296; 4,878,889; and 8,696,542. Other exemplary pelvic condition treating IMD's include the following products produced by American Medical Systems, Inc. of Minnetonka, Minnesota: Monarc™, Sparc™, Elevate™ (Anterior, Posterior, PC Anterior, PC Posterior), Apogee™, Perigee™, Acticon™, MiniArc™, and MiniArc™ Precise.

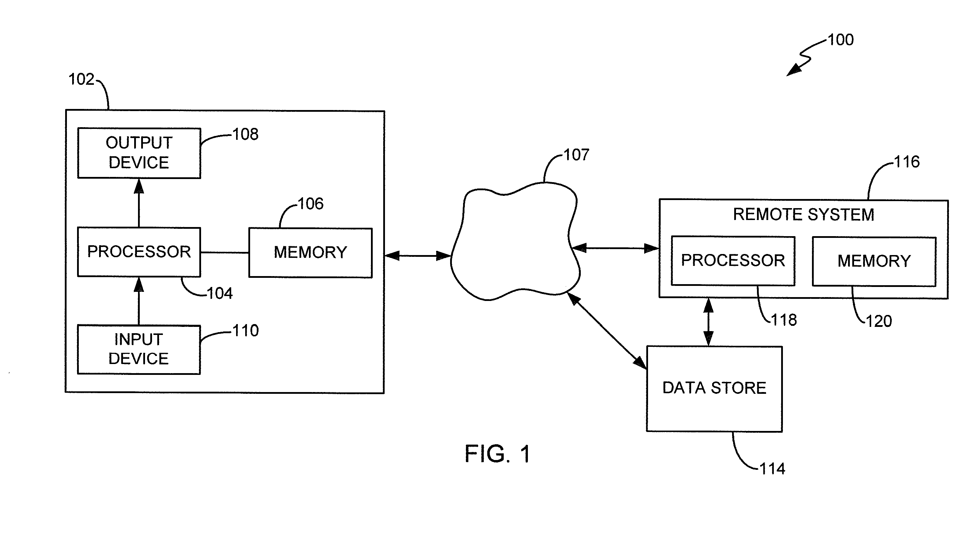

FIG. 1 is a simplified block diagram of a system 100 in accordance with embodiments of the invention that is configured to implement functions, method steps, or operations described herein. In some embodiments, the system 100 includes a plurality of computing devices 102. In some embodiments, the devices 102 are used by the physician responsible for the implantation of an IMD, to input information regarding the IMD, peri-operative complications, post-operative complications, and/or other information, as discussed below. In some embodiments, each computing device 102 may comprise a mobile device, such as a mobile smartphone. The computing device 102 may also take the form of a computer, such as a laptop computer, a desktop computer, a tablet, or the like.

In some embodiments, the device 102 includes one or more processors or central processing units, which are represented by processor 104. In some embodiments, the device 102 includes memory 106 that is accessible by the processor 104. The memory 106 may be local to the device 102, as shown in FIG. 1, and/or located remotely from the device 102. In some embodiments, the memory 106 comprises a non-transitory computer readable medium.

In some embodiments, instructions are stored in a non-transitory computer readable medium, such as the local memory 106 or other computer readable medium that is accessible by the processor 104, which when executed by the processor 104 causes the processor 104 to perform functions, method steps or operations described herein. Such operations may include, for example, receiving data, processing data, communicating data, displaying data, and/or other functions. References to functions and method steps being performed by the device 102 mean that the functions, operations and method steps are performed using the processor 104 executing instructions as described above.

In some embodiments, the device 102 includes conventional data communication circuitry, which is used by the processor 104 to receive and/or transmit data in accordance with conventional wired or wireless communication protocols. Thus, embodiments of the device 102 include the ability to communicate with devices, systems and/or data stores that are local and/or remote from the device 102, using conventional communication protocols. In some embodiments, the device 102 is configured to communicate over a network 107.

In some embodiments, the device 102 includes or is configured to communicate with one or more output devices 108 in accordance with conventional data communication protocols. In some embodiments, the output device 108 includes a display for displaying information to the user of the device 102. In some embodiments, the display 108 is integrated with the device 102, such as when the device 102 is in the form of a mobile phone or other mobile computing device. This display 108 may also be a separate component from the device 102. In some embodiments, the device 102 uses the display 108 to present queries and other information to the user of the device 102.

In some embodiments, the queries are used to prompt the user to input data to the device 102 using one or more input devices 110. The one or more input devices 110 may be integrated with the device 102 or separate therefrom. Embodiments of the input device or devices 110 include a keypad, a speech recognition system, a touchpad, a touchscreen, and/or other conventional device for inputting data.

In some embodiments, the device 102 presents user-selectable options or elements on the display 108 that are selectable by the user using the input device 110 and/or used to enter data using the input device 110. These options or elements may be displayed as buttons, text boxes, check boxes, and/or other conventional display elements. It is understood that the exemplary display screens described herein may include alternative user-selectable options or elements than those depicted in the illustrations.

In some embodiments, the system 100 includes a remote data store 114, such as a server or website. The devices 102 are each configured to communicate with the data store 114 using conventional communication protocols, such as over the network 107, as illustrated in FIG. 1.

In some embodiments, the system 100 includes a remote system 116 that includes a processor 118 and/or memory 120. In some embodiments, the remote system 116 is configured to access information in the data store 114 and process the information. In some embodiments, the remote system 116 is configured to communicate with the plurality of devices 102 using conventional techniques.

In some embodiments, the initial startup routine of the device 102 presents a splash screen showing a desired logo, such as the logo of the healthcare provider, or the logo of the manufacturer of the IMD's being tracked using the device 102. In some embodiments, an additional display of a disclaimer is presented on the display 108 of the device 102 that explains that the user (e.g., a physician) is responsible for the content on the device 102.

In some embodiments, the user of the device, typically a physician responsible for the implantation of an IMD, logs into the device 102 using the input device 110 in accordance with a conventional security protocol. For instance, the user may have a user ID and password that allows access to the application or applications that may be executed on the device 102 to perform the various functions described herein. In some embodiments, the user login may be implemented only using the device 102, or the login information may be verified through a communication through a network 107 to the data store 114 or the remote system 116, which are illustrated in FIG. 1.

In some embodiments, the device 102 collects a patient-ID and a randomly generated unique case-ID that is linked to the patient-ID. In some embodiments, the link between the case-ID and the patient-ID will be stored locally on the device, such as in the memory 106. In some embodiments, the link between the case-ID and the patient-ID is not communicated through the network 107 for remote storage in the data store 114 or the remote system 116. This embodiment prevents one from tracing a case-ID stored in the data store 114 or the system 116 back to an individual patient. Thus, in some embodiments, only the healthcare provider is able to match a specific patient to the case-ID.

In some embodiments, the device 102 presents a login screen on the display 108 and the user is allowed to perform a secure login routine using the input device 110. In some embodiments, first-time users are allowed to create an account by providing various identification information, such as name and email address, as well as a username and password. In some embodiments, the device 102 requires a unique activation code to complete the generation of an account.

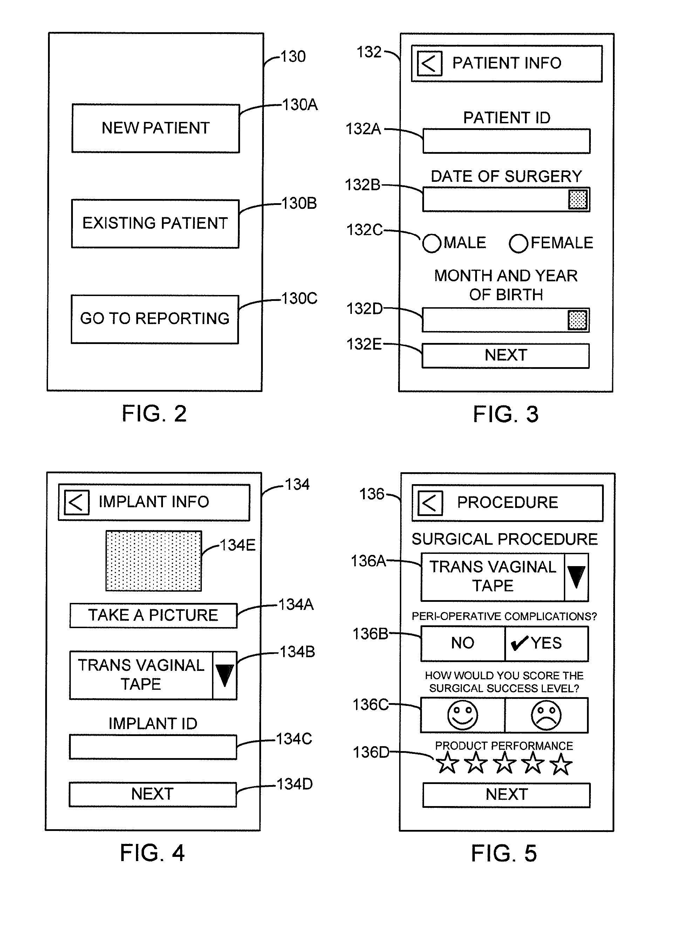

In some embodiments, the device 102 presents a home screen 130 on the display 108, exemplary embodiments of which are illustrated in FIG. 2. The home screen 130 may be presented on the display 108 following, for example, the login of the user. In some embodiments, the home screen 130 presents one or more user-selectable options or elements on the display 108, such as buttons, which may be selected by the user of the device 102 using the input device 110. In some embodiments, the home screen 130 includes an element 130A for adding a new patient, an element 130B for selecting a patient that was previously entered into the system, and/or an element 130C for opening a reporting screen.

If the user selects the new patient element 130A, one or more patient information screens 132 may be displayed on the display 108, exemplary embodiments of which are illustrated in FIG. 3. In some embodiments, each patient information screen 132 includes one or more user-selectable options or elements for entering patient information about the new patient. Exemplary patient information includes the name of the patient, the patient-ID for the patient, the date of birth of the patient, contact information for the patient, and other patient information. In some embodiments, such patient information may be input through the use of drop-down menus, text boxes, or other conventional techniques. In some embodiments, the processor 104 checks the validity of the patient information input by the user before proceeding to another data entry step.

In some embodiments, each patient information screen 132 includes one or more user-selectable options or elements for entering patient information. Exemplary elements include an element for entering a patient-ID (132A), an element for entering a date of the surgery during which an IMD was implanted in the patient (132B), an element for entering the gender of the patient (132C), and an element for entering a date of birth of the patient (132D), for example. Additional elements can be provided on the patient information screen 132 or on other patient information screens, which may be accessed through the selection of element 132E, for example, to add additional patient information. Alternatively, the implant information screen 134, as well as other screens described herein, may be formed larger than the screen size of the display 108, and a conventional feature for scrolling the screen 134 may be provided to accommodate for additional elements for adding patient information.

In some embodiments, the patient-ID is automatically generated by the device 102 or is manually entered by the user. In some embodiments, the device 102 randomly generates a case-ID, links the case-ID with the patient-ID, and stores the case-ID and the patient-ID locally in the memory 106 of the device 102. In some embodiments, all of the patient information is stored locally in the memory 106 of the device 102, and is not communicated outside the healthcare provider. In some embodiments, the patient-ID and the case-ID are stored in the remote data store 114 and/or the memory 120 of the system 116.

In some embodiments, the selection of the existing patient element 130B on the home screen 130 allows the user to select from a list of current patients that have been entered into the device 102. The selection of one of the current patients allows the user to edit the patient information for the selected patient.

In some embodiments, the device 102 presents one or more implant information screens 134 on the display 108, exemplary embodiments of which are illustrated in FIG. 4. In some embodiments, the implant information screen is used to input implant information into the device 102 for a selected or new patient using the input device 110. The user may be prompted to enter the implant information in response to the selection of the element 132E in the patient information screen 132, for example.

Exemplary implant information includes a serial number or implant ID of the IMD implanted in the patient, the manufacturer of the IMD implanted in the patient, an image of the IMD or a label (e.g., a barcode, a Quick Response (QR) code, etc.) corresponding to the IMD, the date of the surgery implanting the IMD in the patient, the type of IMD (e.g., sling, artificial sphincter, electronic stimulator device, penile prosthesis, etc.), a model of the IMD implanted in the patient, and other information regarding the IMD implanted in the patient. In some embodiments, the user is prompted to manually input IMD information for the IMD implanted in the patient into the device 102 using the input device 110. In some embodiments, such information may be input through the use of drop-down menus, text boxes, or other conventional techniques. In some embodiments, the processor 104 checks the validity of the IMD information input by the user before proceeding to another data entry step.

In some embodiments, each implant information screen 134 includes one or more user-selectable options or elements for entering implant information. Exemplary elements include an element for taking a picture (134A), an element for selecting a type of the IMD implanted in the patient (134B), and an element for entering an implant identification for the IMD implanted in the patient (134C). Additional elements can be provided on the implant information screen 134 or on other implant information screens, which may be accessed through the selection of element 134D, for example, to add additional implant information. Alternatively, the implant information screen 134 may be formed larger than the screen size of the display 108, and a conventional feature for scrolling the screen 134 may be provided to accommodate additional elements for adding implant information. The IMD information may be stored in the memory 106, communicated and stored in the data store 114, and/or communicated to the remote system 116 for storage in the memory 120.

In some embodiments, the input device 110 includes a camera, which allows the device 102 to capture an image of the IMD, a label of the IMD, or other information relating to the IMD. In some embodiments, the device 102 prompts the user to capture an image of the IMD or label of the IMD in response to the selection of element 134A. In some embodiments, the captured image is presented as an image 134E on the screen 134. In some embodiments, the device 102 is configured to determine IMD information from the captured image through optical character recognition, a QR code decoder, or other features of the device 102.

In some embodiments, the exemplary element 134B includes a list of IMD's from which the user may select the IMD corresponding to the patient. In some embodiments, the list of IMD's includes one or more of the IMD's listed above that are configured to treat a pelvic condition of the patient. In some embodiments, the element 134B is in the form of a drop-down list, which the user may scroll through using conventional techniques to select the desired IMD. In some embodiments, one or more IMD information fields required by the device 102 are automatically populated in response to the selection of the IMD from the list or element 134B.

In some embodiments, the device 102 presents one or more procedure screens on the display 108, exemplary embodiments of which are illustrated in FIG. 5. In some embodiments, the procedure screen is used to input procedure information into the device 102 relating to the surgical procedure performed on the patient to implant the IMD in the patient. Exemplary patient information includes an identification of the type of IMD implanted in the patient, the date of the surgery, the location where the surgery took place, the name or identification of the physician who performed the surgery, an indication of whether any complications were observed during the surgery, an indication of whether any complications were observed after the surgery, an indication of how successful the surgery was, an indication of how the implanted product or IMD performed, and other procedure information.

In some embodiments, each procedure screen 132 includes one or more user-selectable options or elements for entering procedure information. In some embodiments, the elements include an element 136A identifying the surgical procedure that was performed on the patient. In some embodiments, element 136A is automatically set based on the implant information input by the user, such as through the implant information screen 134 (FIG. 4). In some embodiments, element 136A includes a text box, in which the user may manually input the surgical procedure that was performed to implant the IMD in the patient. Alternatively, the element 136A may include a drop-down menu or other suitable element for entering the surgical procedure information into the device 102 using the input device 110.

In some embodiments, the procedure screen 136 includes an element 136B that prompts the user to indicate whether there were any peri-operative complications (complications that arise during surgery or within 24 hours after surgery). In some embodiments, the element 136B includes a NO button and a YES button. In some embodiments, when the user selects the NO button of the element 136B (FIG. 5), the device 102 prompts the user to indicate his/her evaluation of how the surgery went.

In some embodiments, the user indicates whether the surgical procedure was successful or not through an element 136C, such as through the selection of an appropriate happy face button (successful) or a sad face button (unsuccessful) of the element 136C. Alternative surgery success rating indicators may also be used for element 136C.

In some embodiments, the procedure screen 136 includes an element 136D, which prompts the user to indicate a rating for the product performance. For instance, element 136D may include a rating from poor to excellent in five steps, as shown in FIG. 5.

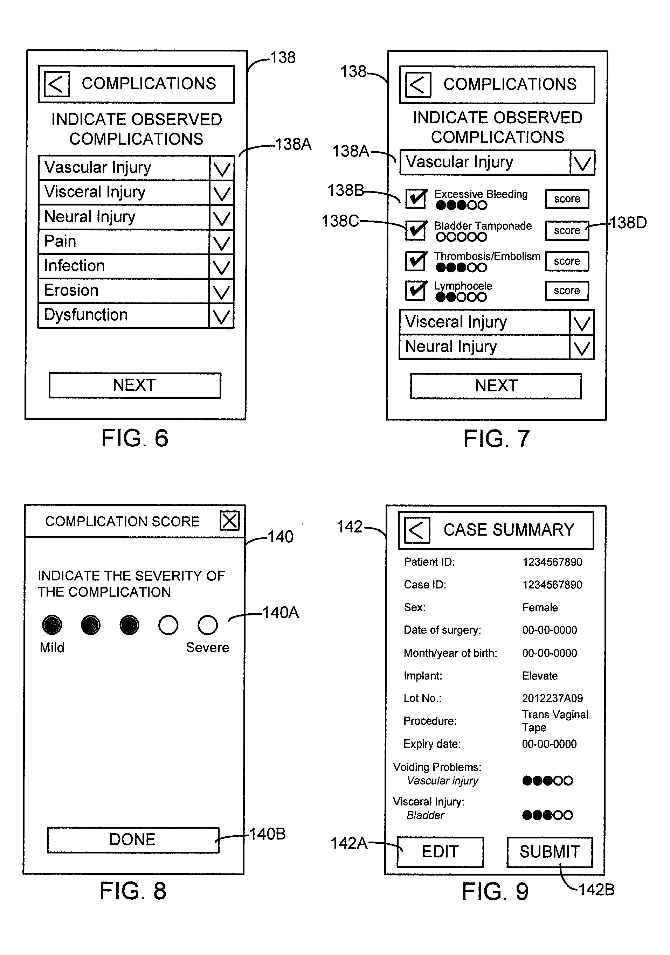

In some embodiments, if the user chooses the YES button of element 136B (FIG. 5) indicating that one or more complications were observed, the device 102 prompts the user to identify the observed complications, and optionally indicate a severity of the observed complications. In some embodiments, the device 102 opens one or more complications screen 138 on the display 108, exemplary embodiments of which are shown in FIG. 6.

In some embodiments, each complications screen 138 includes one or more user-selectable options or elements for entering complication information. Exemplary complication information includes an identification of one or more complications, an identification of a category or group of complications, an identification of specific complications within a group of complications, an indication of a severity of the complication, an indication of the duration of the complication, and other complication related information.

**Table 1**

| Vascular Injury | Visceral Injury |
|---|---|
| Excessive Bleeding | Bladder |
| Bladder Tamponade | Rectal |
| Thrombosis/Embolism | Bowel |
| Lymphocele | Vaginal Wall |
| Neural Injury | Perineum |
| Loss of feeling | Urethra |
| Neuropathy | Ureter |
| Pain | Infection |
| Perineum | Fever (e.g., > 48 hours) |
| Dysuria (painful urination) | Urinary tract |
| Dyspareuna (coital pain) | Excessive discharge |
| Leg pain | Wound dehiscence |
| General pain | Necrosis |
| Exposure | Erosion |
| Vaginal wall | Vaginal wall |
| Cervix | Cervix |
| Peritoneum | Peritoneum |
| Skin | Skin |
| Dysfunction | Bladder |
| Lack of therapeutic effect | Rectum |
| Sexual disorders | Urethra |
| De novo or increased incontinence | |
| De novo or increased vaginal prolapse | |
| Residual urine or bladder retention | |
| Excessive scar formation | |

Exemplary complications that may be listed on the one or more complications screens 138 include those listed in Table 1. In some embodiments, the device 102 lists complications on the one or more complications screens 138 based on the IMD, the patient-ID, and/or the surgical procedure currently being monitored using the device 102. For instance, when the user of the device is viewing screens relating to a sling implantation, the complications that may be listed on the complications screen 138 are limited to those that are predefined as being associated with the sling implantation. In some embodiments, associations between individual or groups of complications and specific IMD's and/or surgical procedures are stored in the memory 106 of the device 102, the data store 114, and/or the memory 120 of the remote system 116.

In some embodiments, such complication information may be input through the use of drop-down menus, text boxes, or other conventional techniques. In some embodiments, each complications screen 138 includes one or more user-selectable options or elements for entering complication information. In some embodiments, the complications screen includes an element 138A that includes a list of available complications that may be selected by the user using the input device 110.

In some embodiments, the complications listed in element 138A may be categorized into groups, which are each identified by a heading and each contains a number of more specific complications that fall under the group. Exemplary groups of complications are provided in Table 1. For instance, "Vascular Injury" is a heading for the complications of "Excessive Bleeding," "Bladder Tamponade," "Thrombosis/Embolism," and "Lymphocele."

In some embodiments, when one of the groups is selected by the user, a sub-menu 138B may open up to present more specific complications to the user, as shown in FIG. 7. For instance, if the user selects the "Vascular Injury" heading button of element 138A, the sub-menu 138B may be presented on the display 108 within the complications screen 138 that lists more specific vascular injury related complications (e.g., excessive bleeding, etc.). In some embodiments, the element 138B includes tick-boxes 138C, which may be selected by the user to indicate the observed complications within the group.

In some embodiments, the device 102 prompts the user to enter a score indicating the severity of an observed complication. For example, the user may be prompted in response to the selection of one of the tick-boxes 138C (FIG. 7). In some embodiments, the device 102 prompts the user to enter a score by displaying a complications score screen 140 on the display 108, as shown in FIG. 8. In some embodiments, the complications score screen 140 includes an element 140A, which may be used to enter a severity score for the complication. In some embodiments, the element 140A includes a series of buttons or other features (e.g., a range of 1-5, etc.) that may be selected by the user to indicate the severity of the complication, as shown in FIG. 8. In some embodiments, after completing the complications score screen 140 for a selected complication, the user is returned to the complications screen 138, such as through the selection of element 140B of the complications score screen 140. The user is then allowed to select additional complications and score the complications as necessary. In some embodiments, a user is allowed to return to the complications score screen 140 corresponding to one of the listed complications by selecting element 138D (FIG. 7) corresponding to the complication.

In some embodiments, after the user inputs one or more complications to the device 102, the user is prompted to report the complications directly to a website or other destination. In some embodiments, the prompt includes a URL for the website where the complications are to be reported. In some embodiments, the device 102 is configured to send the report to the desired location.

In some embodiments, the device 102 presents one or more case summary screens 142 on the display 108, exemplary embodiments of which are illustrated in FIG. 9. In some embodiments, the case summary screen 142 is presented after the user has completed the input of any observed complications on the complications screen 138 (FIG. 7), or when the user indicates that there are no observed complications. In some embodiments, the case summary screen 142 provides a summary of the data input through the patient information screen 132, the implant information screen 134, the procedure screen 136, and/or the complications screen 138.

In some embodiments, the device 102 allows the user to edit the input data presented on the case summary screen 142 by selecting one or more of the entries presented in the case summary screen 142. In some embodiments, such a selection causes the device 102 to return to the data input screen corresponding to the selection. For instance, if the user selects implant data to edit, the device 102 opens the corresponding implant information screen 134 where the user is allowed to make modifications. In some embodiments, the case summary screen 142 includes an element 142A, which, when selected by the user, allows the user to edit the input data.

In some embodiments, the case summary screen 142 includes an element 142B, which prompts the user to submit the information presented on the case summary screen 142 if the information appears to be correct. In some embodiments, when the user selects the element 142B to submit the information, a data set is uploaded through the network 107 to the remote data store 114 (e.g., server or cloud-based server), or another location, such as the remote system 116. In some embodiments, only information relating to the implant and the case-ID are uploaded. That is, in some embodiments, the corresponding patient-ID and/or patient identification information is not uploaded to the remote locations through the network 107.

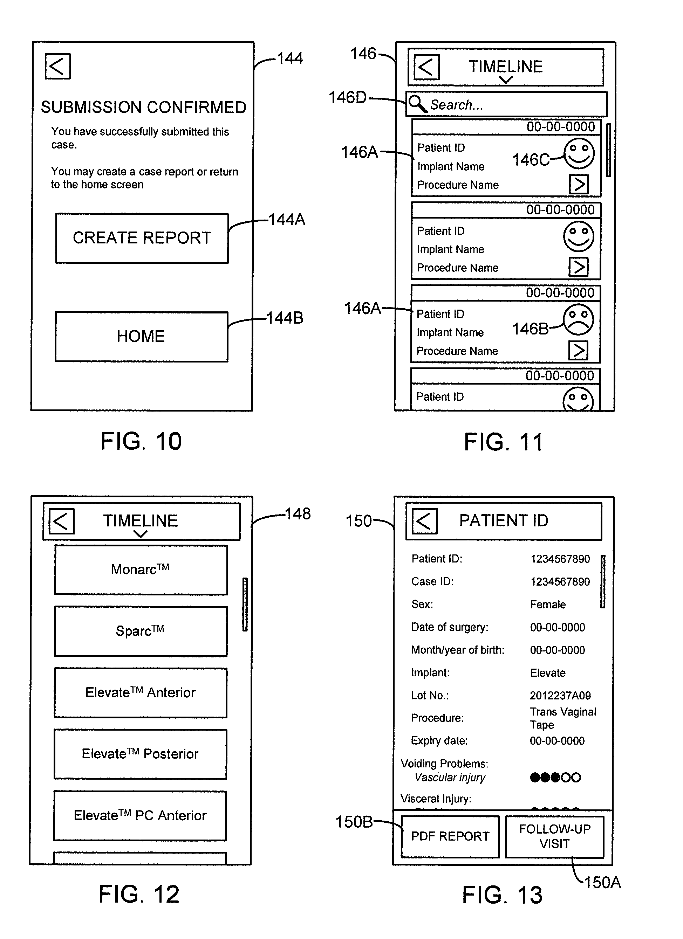

In some embodiments, if the user successfully uploads the case summary and related information, he/she is notified accordingly by the device 102 on the display 108, such as through the presentation of a submission confirmation screen 144 on the display 108, exemplary embodiments of which are illustrated in FIG. 10. In some embodiments, the submission confirmation screen 144 includes an element 144A, which may be selected by the user to create a report for the particular implant and/or patient in an electronic format (e.g., a pdf document). In some embodiments, the submission confirmation screen 144 includes an element 144B, which the user may select to return to the home screen 130 or other screen.

In some embodiments, the device 102 presents one or more timeline screens 146, exemplary embodiments of which are illustrated in FIG. 11. The one or more timeline screens 146 provide a visualization of a group of implantations that the healthcare provider has performed. In some embodiments, the timeline screens 146 include entries 146A, each of which includes information relating to the performed procedure that has been stored in the memory 106 of the device 102, or in memory that is accessible by the device 102, such as the data store 114 or the memory 120 of the remote system 116, for example. In some embodiments, the entries 146A each include patient information, implant information, procedure information, observed complications, and/or other information that has been input regarding the performed procedure. In some embodiments, the entries 146A of the timeline screen 146 are sorted based on the date of the surgery. Thus, the user of the device always sees his/her most recent case first.

In some embodiments, the entries 146A corresponding to surgical procedures where complications were observed include a notification or an alert that complications were observed. For instance, an icon 146B may be placed in the entry 146A to provide a visualization that complications were observed in the surgical procedure corresponding to the entry 146A. The user of the device 102 may scroll through the entries 146A and quickly identify the surgical procedures in which complications were observed by viewing the icons 146B. In some embodiments, the entries 146A include icons 146C, which indicate that no complications were observed during and/or following the surgical procedure.

In some embodiments, the timeline screen 146 includes a search window 146D that allows the user to quickly access an individual case/patient through the entry of a search query using the input device 110. In some embodiments, the search query input in the search window 146D may be used to filter the timeline of entries 146A presented on the timeline screen 146. For instance, a user may filter the entries 146A to include only entries 146A for a specific surgical procedure or a specific implant. In some embodiments, a filter menu may be provided for setting various parameters of the filter. For instance, a filter screen 148 may be provided that includes a list of IMD's, as illustrated in FIG. 12. In some embodiments, when the user selects one of the IMD's in the list, the timeline screen 146 is presented listing only entries 146A corresponding to the selected IMD.

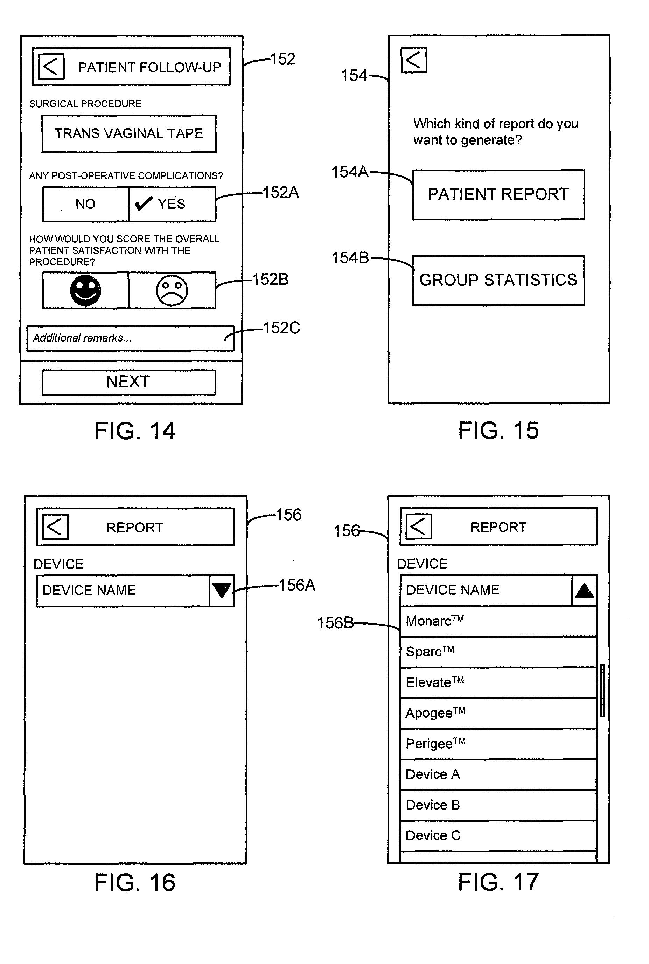

In some embodiments, when the user selects a specific case, such as a case presented as an entry 146A in the timeline screen 146, the device 102 shows a summary of all or a portion of the information that was collected for that specific case or surgical procedure. In some embodiments, the device 102 presents a summary screen 150 listing the information, embodiments of which are illustrated in FIG. 13. In some embodiments, the user may enter new information for the selected case, such as information obtained from a follow-up visit from the patient by selecting element 150A presented on the summary screen 150. In some embodiments, the selection of element 150A causes the device 102 to open a patient follow-up screen 152 on the display 108, embodiments of which are illustrated in FIG. 14.

In some embodiments, the patient follow-up screen 152 allows the user to enter new information regarding complications that have occurred in the period between the operation and the patient's most recent visit to the clinic. In some embodiments, the follow-up screen 152 includes an element 152A that prompts the user to indicate whether any post-operative complications have been observed. If the user indicates that post-operative complications have been observed by selecting the YES button of element 152A, the device 102 opens a post-operative complications screen, which lists various post-operative complications. In some embodiments, the post-operative complications screen allows the user to select the observed post-operative complications and indicate a severity of the complications, in a similar manner as that described above with regard to the complications screen 138.

In some embodiments, the patient follow-up screen 152 includes an element 152B, which prompts the user to score the overall patient satisfaction with the procedure. In some embodiments, the element 152B may include elements that indicate whether the patient was satisfied or dissatisfied with the procedure, such as the happy face and sad face buttons shown in FIG. 14, buttons that provide a satisfaction score (e.g., 1-5), or other elements that may be used to indicate the patient's level of satisfaction with the procedure. In some embodiments, the patient follow-up screen 152 includes an element 152C, in which the user may enter additional remarks regarding the patient follow-up.

In some embodiments, the summary screen 150 for a selected case includes an element 150B, which the user may select to generate a report regarding the current case. In some embodiments, the device 102 is configured to provide the user with one or more options of emailing the report, storing the report in the memory 106, communicating the report for storage in the remote data store 114, communicating the report to the remote system 116 for storage in the memory 120, or other option.

In some embodiments, the device 102 is configured to present a report screen 154 on the display 108, embodiments of which are illustrated in FIG. 15. In some embodiments, the report screen 154 includes an element 154A, which the user may select to generate a patient report. In some embodiments, the selection of element 154A by the user causes the device 102 to allow the user to select a desired patient for which a report should be generated.

In some embodiments, the report screen 154 includes an element 154B, which when selected by the user allows the user to generate a group statistics report. In some embodiments, the selection of the element 154B by the user causes the device 102 to open a device report screen 156 on the display 108, embodiments of which are illustrated in FIG. 16. In some embodiments, the device report screen 156 includes an element 156A listing various IMD's that the user may select to generate a report on the selected IMD. In some embodiments, the element 156A is in the form of a drop-down menu, the selection of which opens a sub-menu 156B, as shown in the report screen 156 illustrated in FIG. 17. In some embodiments, the sub-menu 156B lists the various IMD's that are available for selection by the user.

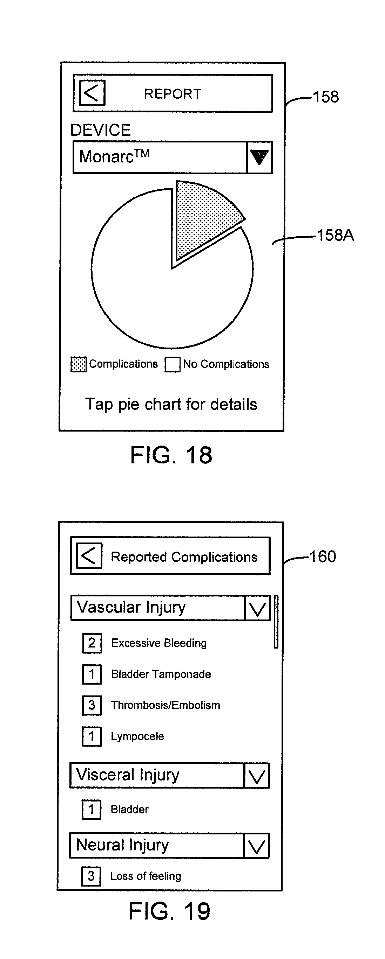

In some embodiments, the selection of one of the IMD's listed in the sub-menu 156B of the report screen 156 causes the device 102 to open a report screen 158 on the display 108, embodiments of which are illustrated in FIG. 18. In some embodiments, the report screen 158 includes an element 158A, which illustrates a measure of a frequency at which complications are observed relating to the IMD. In some embodiments, the element 158 includes a graphical illustration of the frequency of observed complications with regard to the IMD, such as a pie chart (shown), a bar graph, or other graphical illustration. In some embodiments, the element 158A may be presented on the same screen 156, from which the user selected the IMD.

In some embodiments, the user may select the element 158A or other element on the report screen 158 to access details regarding the reported complications relating to the IMD. In some embodiments, such a selection opens a reported complications screen 160 on the display 108, embodiments of which are illustrated in FIG. 19. In some embodiments, the reported complications screen 160 provides a list of the complications relating to the selected IMD that have been observed. In some embodiments, screen 160 provides a total of the number of times each complication was observed for the IMD, indicates a frequency at which each of the complications has been observed, or provides another measure of the complications relating to the IMD that have been observed. In some embodiments, the complications listed on the reported complications screen 160 are organized in a manner similar to that described above with regard to the complications screen 138. In some embodiments, the reported complications screen 160 only lists the complications that were actually reported by the device 102. That is, the device 102 filters out non-reported complications.

Some embodiments of the invention are directed to a method of monitoring complications relating to a plurality of medical devices, which are each implanted in one of a plurality of patients. Embodiments of the method include one or more method steps or functions described above. In some embodiments of the method, the medical devices or IMD's are configured to treat a pelvic condition of a patient, such as those described above. In some embodiments, the medical devices are selected from the medical devices described above, such as a sling, an artificial sphincter, a penile prosthesis, or an electronic stimulator device.

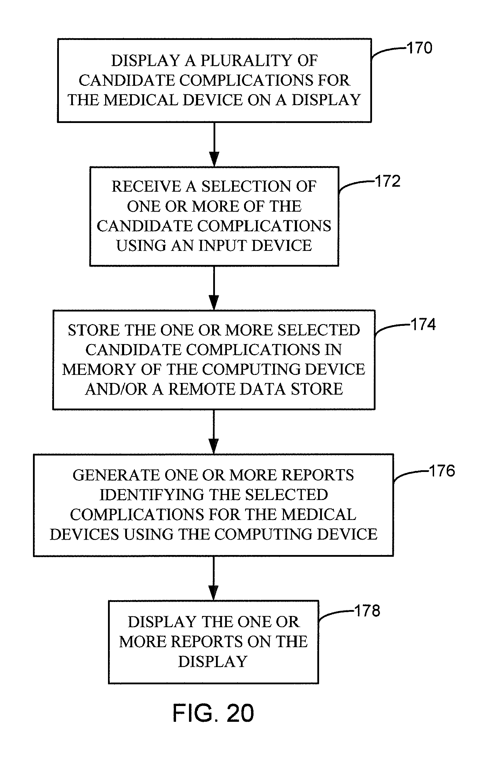

FIG. 20 is a flowchart illustrating the method in accordance with some embodiments of the invention. In some embodiments of the method, complication information is entered for each of the implanted medical devices in a computing device 102. In some embodiments of this method step, the device 102 displays a plurality of candidate complications for the medical device on a display 108, as indicated at 170. In some embodiments, step 170 is carried out as described above with reference to the complications screen 138 illustrated in FIGS. 6 and 7. In some embodiments, the candidate complications include one or more peri-operative complications and/or one or more post-operative complications. In some embodiments, the candidate complications are selected from those described above, including those listed in table 1. In some embodiments, the display of the candidate complications provided in step 170 identifies group of candidate complications by a group heading, as described above. At 172, a selection of one or more of the candidate complications is received using an input device 110. Embodiments of step 172 include one or more embodiments described above with regard to the selection of the candidate complications using the input device 110. At 174, the one or more selected candidate complications are stored in memory 106 of the computing device 102 and/or a remote data store. In some embodiments, the remote data store includes the data store 114 and/or the memory 120 of the remote system 116, which are shown in FIG. 1 and comprise a non-transitory computer readable medium. Embodiments of step 174 and other steps described herein are carried out in accordance with one or more embodiments and techniques described above using the device 102.

In some embodiments of the method, one or more reports are generated that identify the selected complications for the medical devices using the computing device 102, as indicated at 176. Embodiments of step 176 may be carried out in accordance with the method and techniques described above using the device 102 including those described with reference to FIGS. 13 and 15-19.

At 178 of the method, the one or more reports are presented on the display 108. Step 178 may be carried out in accordance with one or more methods and techniques described above using the device 102. For instance, the device 102 may present various reports on the display 108 including a summary screen 150 (FIG. 13), a report screen 158 (FIG. 18), and/or a report screen 160 (FIG. 19), for example.

In some embodiments, the entering of complication information into the device 102 for each of the implanted medical devices includes entering a score indicating the severity of the complication for each of the selected candidate complications received at step 172 using the device 102.

In some embodiments, the medical devices or IMD's include different types of IMD's. In some embodiments, step 176 includes the generation of a report that includes a summary of the selected complications for each of the different types of medical devices.

In some embodiments of the method, patient information is entered for each of the patients in the computing device 102 using the input device 110. Embodiments of the patient information include those described above. In some embodiments of steps 176 and 178, the reports generated and/or displayed on the device 102 include a patient report or summary for one of the patients that includes the patient information and the selected complications for the medical device implanted in the patient, as described above with reference to the patient or case summary screen 142 shown in FIG. 9.

In some embodiments of steps 176 and/or 178, a report is generated and/or displayed that lists a plurality of surgeries that were performed to implant the medical devices on the display 108, as discussed above with regard to the timeline screen 146 shown in FIG. 11. In some embodiments, the report includes an icon 146B or 146C indicating whether one or more complications relating to at least one of the surgeries were observed.

Some embodiments of the invention are directed to one or more embodiments of the computing device 102 described above. In some embodiments, the computing device 102 includes the processor 104, memory 106 comprising a non-transitory computer readable medium, a display 108, and an input device 110, which are shown in FIG. 1. In some embodiments, instructions are stored in the non-transitory computer readable medium 106, which when executed by the processor 104 causes the processor 104 to perform operations for monitoring complications relating to medical device implantations in a plurality of patients.

In some embodiments, the operations cause the device 102 to perform the method illustrated in the flowchart of FIG. 20. Thus, in some embodiments, the operations performed by the processor 104 in response to the execution of the instructions stored in the memory 106 include entering complication information for each of the implanted medical devices comprising: displaying (170) a plurality of candidate complications for the medical device on a display 108; receiving (172) a selection of one or more candidate complications corresponding to the medical device from an input device 110; and storing (174) the one or more selected candidate complications in at least one of the memory 106 of the computing device and a remote data store (e.g., data store 114 or memory 120). In some embodiments, the operations include generating (176) one or more reports identifying the selected complications for the medical devices, and displaying (178) the report on the display 108.

In some embodiments, the medical devices are configured to treat a pelvic condition of the patient selected from the group consisting of urinary incontinence, pelvic organ prolapse, fecal incontinence, urine retention, sexual dysfunction, and pelvic pain. In some embodiments, the medical devices are each selected from the group consisting of a sling, an artificial sphincter, a penile prosthesis, and an electronic stimulator device.

In some embodiments, the input device 110 includes at least one input device selected from the group consisting of a keypad, a speech recognition system, a touchpad, and a touchscreen.

In some embodiments, the candidate complications are selected from the group consisting of vascular injury, visceral injury, neural injury, pain, infection, dysfunction, excessive bleeding, bladder cap tamponade, thrombosis, embolism, lymphocele, visceral injury to the bladder, visceral rectal injury, visceral bowel injury, visceral injury to the vaginal wall, visceral injury to the perineum, visceral injury to the urethra, visceral injury to the ureter, loss of feeling, neuropathy, perineum pain, dysuria, dyspareunia, leg pain, genital pain, fever, urinary tract infection, wound dehiscence, necrosis, increased incontinence, increased vaginal prolapse, and urinary tension.

In some embodiments, the operation of entering complication information for each of the implanted medical devices comprises entering a score for each of the selected candidate complications using the input device 102. In some embodiments, the score is indicative of a severity of the complication.

In some embodiments, the plurality of medical devices comprises first and second medical devices, and generating one or more reports identifying the selected complications for the medical devices using the computing device 102 comprises generating a report comprising a summary of the received selected complications corresponding to the first medical devices, and generating a report comprising a summary of the received selected complications corresponding to the second medical devices.

In some embodiments, the operations performed by the processor 104 in response to the execution of instructions stored on the computer readable medium include entering patient information for each of the patients in the computing device 102 using the input device 110, the patient information including at least two pieces of information selected from the group consisting of an identification of the patient, an identification of a surgical procedure performed on the patient to implant the medical device, and an identification of the medical device implanted in the patient. In some embodiments, the operation of generating one or more reports identifying the selected complications for the medical devices comprises generating a patient report, such as the patient summary screen 150 (FIG. 13) for one of the patients that includes the patient information and the selected complications corresponding to the medical device implanted in the patient.

Some embodiments of the operations comprise generating a timeline screen 146 listing a plurality of surgeries that were performed to implant the medical devices on the display 108, wherein the listing includes an alert icon 146B indicating that one or more complications relating to at least one of the surgeries were observed.

Although the present invention has been described with reference to preferred embodiments, workers skilled in the art will recognize that changes may be made in form and detail without departing from the scope of the invention.

## Claims

1. A method of monitoring complications relating to a plurality of medical devices, which are each implanted in one of a plurality of patients, the method comprising:
entering complication information for each of the implanted medical devices in a computing device (102) comprising:
displaying (170) a plurality of candidate complications for the medical device on a display (108) of the computing device;
receiving (172) a selection of one or more of the candidate complications using an input device (110) of the computing device; and
storing (174) the one or more selected candidate complications in at least one of memory of the computing device and a remote data store (106, 114, 120); and
generating (176) one or more reports identifying the selected complications for the medical devices using the computing device; and
displaying (178) the one or more reports on the display.

2. The method of claim 1, wherein the medical devices are configured to treat a pelvic condition of the patient selected from the group consisting of urinary incontinence, pelvic organ prolapse, fecal incontinence, urine retention, sexual dysfunction, and pelvic pain.

3. The method of claim 2, wherein the medical devices are each selected from the group consisting of a sling, an artificial sphincter, a penile prosthesis, and an electronic stimulator device.

4. The method of claim 1, 2, or 3, wherein the candidate complications include at least one of a peri-operative complication and a post-operative complication.

5. The method of claim 4, wherein the candidate complications are selected from the group consisting of vascular injury, visceral injury, neural injury, pain, infection, dysfunction, excessive bleeding, bladder tamponade, thrombosis, embolism, lymphocele, visceral injury to the bladder, visceral rectal injury, visceral bowel injury, visceral injury to the vaginal wall, visceral injury to the perineum, visceral injury to the urethra, visceral injury to the ureter, loss of feeling, neuropathy, perineum pain, dysuria, dyspareunia, leg pain, general pain, fever, urinary tract infection, wound dehiscence, necrosis, increased incontinence, increased vaginal prolapse, and urine retention.

6. The method of claim 5, wherein displaying a plurality of candidate complications for the medical device on a display of a computing device comprises identifying groups of candidate complications by a group heading.

7. The method of any one of claims 1 to 6, wherein entering complication information for each of the implanted medical devices comprises entering a score for each of the selected candidate complications using the input device, wherein the score is indicative of a severity of the complication.

8. The method of any one of claims 1 to 7, wherein the plurality of medical devices comprises first and second medical devices, and generating one or more reports identifying the selected complications for the medical devices using the computing device comprises generating a report comprising a summary of the selected complications for the first medical devices, and generating a report comprising a summary of the selected complications for the second medical devices.

9. The method of any one of claims 1 to 8, further comprising entering patient information for each of the patients in the computing device using the input device, the patient information including at least two pieces of information selected from the group consisting of an identification of the patient, an identification of a surgical procedure performed on the patient to implant the medical device, and an identification of the medical device implanted in the patient.

10. The method of claim 9, wherein generating one or more reports identifying the selected complications for the medical devices using the computing device comprises generating a patient report for one of the patients that includes the patient information and the selected complications for the medical device implanted in the patient.

11. The method of any one of claims 1 to 10, further comprising generating a timeline screen listing a plurality of surgeries that were performed to implant the medical devices on the display, wherein the listing includes an alert icon indicating that one or more complications relating to at least one of the surgeries were observed.

12. A computing device (102) comprising:
a processor (104);
memory (106) comprising a non-transitory computer readable medium;
a display (108);
an input device (110); and
instructions stored in the non-transitory computer readable medium, which when executed by the processor causes the processor to perform operations for monitoring complications relating to medical device implantations in a plurality of patients comprising:
entering complication information for each of the implanted medical devices comprising:
displaying (170) a plurality of candidate complications for the medical device on the display;
receiving (172) a selection of one or more candidate complications corresponding to the medical device from the input device; and
storing (174) the one or more selected candidate complications in at least one of the memory of the computing device and a remote data store; and
generating (176) one or more reports identifying the selected complications for the medical devices; and
displaying (178) the report on the display.

13. The computing device of claim 12, wherein the medical devices are configured to treat a pelvic condition of the patient selected from the group consisting of urinary incontinence, pelvic organ prolapse, fecal incontinence, urine retention, sexual dysfunction, and pelvic pain.

14. The computing device of claim 12 or 13, wherein the medical devices are each selected from the group consisting of a sling, an artificial sphincter, a penile prosthesis, and an electronic stimulator device.

15. The computing device of claim 12, 13, or 14, wherein the candidate complications are selected from the group consisting of vascular injury, visceral injury, neural injury, pain, infection, dysfunction, excessive bleeding, bladder tamponade, thrombosis, embolism, lymphocele, visceral injury to the bladder, visceral rectal injury, visceral bowel injury, visceral injury to the vaginal wall, visceral injury to the perineum, visceral injury to the urethra, visceral injury to the ureter, loss of feeling, neuropathy, perineum pain, dysuria, dyspareunia, leg pain, general pain, fever, urinary tract infection, wound dehiscence, necrosis, increased incontinence, increased vaginal prolapse, and urine retention.
